(19) Europäisches Patentamt
European Patent Office
Office européen des brevets

(11) **EP 2 482 113 B1**

(12) **EUROPÄISCHE PATENTSCHRIFT**

(45) Veröffentlichungstag und Bekanntmachung des Hinweises auf die Patenterteilung:
**27.12.2017   Patentblatt 2017/52**

(51) Int Cl.:
*G02B 21/00* *(2006.01)*          *A61B 5/00* *(2006.01)*
*G01B 9/02* *(2006.01)*           *A61B 3/13* *(2006.01)*
*A61B 3/12* *(2006.01)*

(21) Anmeldenummer: **12165225.9**

(22) Anmeldetag: **10.10.2007**

(54) **Operationsmikroskop mit OCT-System**

Operation microscope with OCT system

Microscope d'opération doté d'un système OCT

(84) Benannte Vertragsstaaten:
**DE ES FR GB IT**

(30) Priorität: **06.11.2006   DE 102006052513**
**24.04.2007   DE 102007019680**

(43) Veröffentlichungstag der Anmeldung:
**01.08.2012   Patentblatt 2012/31**

(62) Dokumentnummer(n) der früheren Anmeldung(en) nach Art. 76 EPÜ:
**07019795.9 / 1 918 755**

(73) Patentinhaber: **Carl Zeiss Meditec AG**
**07745 Jena (DE)**

(72) Erfinder:
• **Reimer, Peter**
**73479 Ellwangen (DE)**
• **Hauger, Christoph**
**73431 Aalen (DE)**
• **Abele, Alfons**
**73527 Schwäbisch Gmünd (DE)**
• **Seeßelberg, Markus**
**73431 Aalen (DE)**

(74) Vertreter: **Carl Zeiss AG - Patentabteilung**
**Patentabteilung / CLP-P**
**Carl-Zeiss-Strasse 22**
**73447 Oberkochen (DE)**

(56) Entgegenhaltungen:
EP-A- 0 697 611      EP-A- 0 815 801
EP-A- 1 326 117      DE-U1- 9 415 219

**Beschreibung**

Die Erfindung betrifft ein Ophthalmo-Operationsmikroskop mit einem Mikroskop-Hauptobjektiv, mit einem Beobachtungsstrahlengang, der das Mikroskop-Hauptobjektiv durchsetzt, und mit einem OCT-System zur Aufnahme von Bildern eines Objektbereichs, wobei das OCT-System einen OCT-Abtaststrahlengang umfasst, der über eine Scanspiegelanordnung zum Objektbereich geführt ist.

Ein Operationsmikroskop der eingangs genannten Art ist aus der EP 0 697 611 A2 bekannt. Dieses Operationsmikroskop enthält ein OCT-System, das einen Abtaststrahlengang aus kurzkohärenter Laserstrahlung erzeugt. Das OCT-System enthält eine Analyseeinheit zur Auswertung von Interferenzsignalen. Es umfasst eine Einrichtung zum Scannen des OCT-Abtaststrahlengangs mit zwei Scanspiegeln, die um zwei Bewegungsachsen verstellt werden können. Der OCT-Abtaststrahlengang in dem Operationsmikroskop ist über einen Teilerspiegel in den Beleuchtungsstrahlengang des Operationsmikroskops eingekoppelt. Er wird mit diesem durch das Mikroskop-Hauptobjektiv hindurch zum Objektbereich gelenkt.

Ein OCT-System erlaubt mittels optischer Kohärenztomographie die nichtinvasive Darstellung und Messung von Strukturen innerhalb eines Gewebes. Als optisches bildgebendes Verfahren ermöglicht die optische Kohärenztomographie insbesondere Schnitt- oder Volumenbilder von biologischem Gewebe mit Mikrometerauflösung zu erzeugen. Ein entsprechendes OCT-System umfasst eine Quelle für zeitlich inkohärentes und räumlich kohärentes Licht mit einer Kohärenzlänge $l_c$, die einem Probenstrahlengang und einem Referenzstrahlengang zugeführt wird. Der Probenstrahlengang ist auf das zu untersuchende Gewebe gerichtet. Laserstrahlung, die aufgrund von Streuzentren im Gewebe in den Probenstrahlengang zurückgestrahlt wird, überlagert das OCT-System mit Laserstrahlung aus dem Referenzstrahlengang. Durch die Überlagerung entsteht ein Interferenzsignal. Aus diesem Interferenzsignal lässt sich die Position von Streuzentren für die Laserstrahlung im untersuchten Gewebe bestimmen.

Für OCT-Systeme ist das Bauprinzip des "Time-Domain OCT" und des "Fourier-Domain OCT" bekannt.

Der Aufbau eines "Time-Domain OCT" ist beispielsweise in der US 5,321,501 anhand von Fig. 1a auf Sp. 5, Z. 40 - Sp. 11, Z. 10 beschrieben. In einem solchen System wird die optische Weglänge des Referenzstrahlenganges über einen schnell beweglichen Referenzspiegel fortlaufend variiert. Das Licht aus Proben- und Referenzstrahlengang wird auf einem Photodetektor überlagert. Wenn die optischen Weglängen von Proben- und Referenzstrahlengang übereinstimmen, entsteht auf dem Photodetektor ein Interferenzsignal.

Ein "Fourier-Domain OCT" ist beispielsweise in der WO 2006/10544 A1 erläutert. Um die optische Weglänge eines Probenstrahlenganges zu vermessen, wird wiederum Licht aus dem Probenstrahlengang Licht aus einem Referenzstrahlengang überlagert. Im Unterschied zu einem "Time-Domain OCT" wird jedoch für eine Messung der optischen Weglänge des Probenstrahlenganges das Licht aus Proben- und Referenzstrahlengang nicht direkt einem Detektor zugeführt, sondern zunächst mittels eines Spektrometers spektral zerlegt. Die so erzeugte spektrale Intensität des überlagerten Signals aus Proben- und Referenzstrahlengang wird dann mit einem Detektor erfasst. Durch Auswerten des Detektorsignals kann wiederum die optische Weglänge des Probenstrahlenganges ermittelt werden.

In der EP 0697611 A2 ist ein Operationsmikroskop mit einem OCT-System und einem Autofokussystem offenbart. Mit Hilfe des OCT-Systems wird die Lage transparenter Strukturen im Vorderkammerbereich eines Auges (zum Beispiel Kapselsack) in axialer Richtung ermittelt. Anschließend wird ein Steuersignal an das Autofokussystem geleitet, so dass das Operationsmikroskop auf die ermittelte Lage der transparenten Struktur fokussiert wird.

Aus der EP 1 326 117 A1 ist ein Operationsmikroskop mit einem Ophthalmoskie-Vorsatzmodul bekannt, das eine Ophthalmoskopierlupe eines Auges sowie eine Fokus-Optik zur Fokussierung der Beobachtungsstrahlengänge des Operationsmikroskops in einer Zwischenbildebene, in welche die Ophthalmoskopierlupe ein seitenverkehrtes Bild des Fundus' wirft, umfasst. Das Operationsmikroskop liefert einem Operateur somit ein visuelles Bild des Fundus' eines Patientenauges, Ein OCT-System ist in dieser Druckschrift nicht offenbart.

Aus dem deutschen Gebrauchsmuster G 94 15 219 ist ein weiteres Operationsmikroskop mit Ophthalmoskopierlupe zur Betrachtung eines Fundus, jedoch ohne OCT-System bekannt.

In der EP 0 815 801 A2 ist ein Operationsmikroskop für neurologische Anwendungen mit einem OCT-System und einem Mehrfach-Koordinaten Manipulator ("multi-coordinate manipulator MCM") offenbart. Die mit dem OCT-System gewonnenen Daten lassen sich an einem Monitor darstellen oder mit vorab gespeicherten diagnostischen Bilddaten, zum Beispiel einer CT-Aufnahme, korrelieren oder zur Unterscheidung zwischen einem Gehirntumor und normalen Gehirngewebe verwenden. Ophthalmologische Anwendungen sind in der EP 0 815 801 A nicht offenbart.

Aufgabe der Erfindung ist es, ein kompakt aufgebautes Ophthalmo-Operationsmikroskop bereitzustellen, das die Aufnahme von OCT-Schnittbildern des menschlichen Auges sowohl im vorderen Abschnitt des Auges als auch in dessen Augenhintergrund ermöglicht, wobei der OCT-Abtaststrahlengang ohne Intensitätsverluste zu einer OCT-Abtastebene geführt werden kann.

Die Aufgabe wird durch ein Ophthalmo-Operationsmikroskop nach Anspruch 1 gelöst. Die Ophthalmoskopierlupe ermöglicht, den OCT-Abtaststrahlengang auf dem Hintergrund eines Patientenauges zu bündeln, um den Augenhintergrund abzutasten und gleichzeitig diesen im Okulareinblick durch die optischen Beobachtungsstrahlengänge des Operationsmikroskops sichtbar zu machen. Ophthalmoskopierlupe und Reduzierlinse sind in einem Ophthalmoskopie-Vorsatzmodul angeordnet, welches in den OCT-Abtaststrahlengang und in den Beobachtungsstrahlengang ein- und ausgeschwenkt werden kann. Auf diese Weise kann mit dem Ophthalmo-Operationsmikroskop sowohl der vordere Abschnitt eines Patientenauges als auch dessen Augenhintergrund mit OCT-Strahlung untersucht werden.

In Weiterbildung der Erfindung ist zwischen der Scanspiegelanordnung und dem Mikroskop-Hauptobjektiv ein Optikelement vorhanden, das den aus der Scanspiegelanordnung austretenden OCT-Abtaststrahlengang bündelt und in einen Strahlengang überführt, der das Mikroskop-Hauptobjektiv durchsetzt.

In Weiterbildung der Erfindung ist das Optikelement zwischen Scanspiegelanordnung und Mikroskop-Hauptobjektiv als bewegliche Linseneinheit ausgeführt. Auf diese Weise können unterschiedliche Schnittebenen eines menschlichen Auges mit OCT-Strahlung abgetastet werden.

In Weiterbildung der Erfindung ist dieses Optikelement in einer Linsenwechseleinrichtung aufgenommen. Auf diese Weise wird ein schnelles Hin- und Herschalten zwischen unterschiedlichen Abtastebenen für OCT-Strahlung in einem Objektbereich möglich.

In Weiterbildung der Erfindung ist das Optikelement als Zoomsystem mit variabler Brennweite ausgeführt. Auf diese Weise wird eine stufenlose Variation von in einem Patientenauge untersuchten Schnittebenen mit OCT-Strahlung ermöglicht.

In Weiterbildung der Erfindung sind Mittel zur Einstellung der Brennweite des Hauptobjektivs vorhanden. Auf diese Weise können mit dem Ophthalmo-Operationsmikroskop unterschiedliche Ebenen eines Objektbereichs untersucht werden, ohne dass dabei das Operationsmikroskop verlagert werden muss.

In Weiterbildung der Erfindung umfasst die Scanspiegelanordnung für das Scannen des OCT-Abtaststrahlengangs einen ersten Scanspiegel. Vorzugsweise ist zusätzlich ein zweiter Scanspiegel vorhanden, wobei der erste Scanspiegel um eine erste Drehachse bewegt werden kann und der zweite Scanspiegel um eine zweite Drehachse bewegt werden kann, und wobei die erste Drehachse und die zweite Drehachse seitlich versetzt in einem rechten Winkel zueinander stehen. Auf diese Weise kann ein Objektbereich mit einem zueinander senkrecht verlaufenden Rastermuster abgetastet werden.

In Weiterbildung der Erfindung umfasst das OCT-System einen Lichtleiter, der einen Lichtaustrittsabschnitt für den OCT-Abtaststrahlengang hat, dem Mittel zum Bewegen zugeordnet sind. Auf diese Weise kann das OCT-System für die Verwendung von OCT-Strahlung unterschiedlicher Wellenlänge angepasst werden.

Vorteilhafte Ausführungsformen der Erfindung sind in den Figuren dargestellt und werden nachfolgend beschrieben.

Es zeigen:

Fig. 1          ein Ophthalmo-Operationsmikroskop mit Ophthalmoskopie-Vorsatzmodul und einem ersten OCT-System;

Fig. 2          das Ophthalmo-Operationsmikroskop aus Fig. 1 mit einem zweiten OCT-System, wobei das Ophthalmoskopie-Vorsatzmodul in den Beobachtungsstrahlengang des Ophthalmo-Operationsmikroskops eingeschwenkt ist;

Fig. 3          einen Schnitt des Mikroskop-Hauptobjektivs entlang der Linie III - III aus Fig. 1;

Fig. 4          einen Abschnitt des Ophthalmo-Operationsmikroskops mit einem ersten und einem zweiten OCT-System;

Fig. 5          eine Intensitätsverteilung des aus dem Lichtleiter des OCT-Systems im Operationsmikroskop austretenden OCT-Abtastlichtstrahls; und

Fig. 6          eine Intensitätsverteilung des OCT-Abtastlichtstrahls in der OCT-Abtastebene im Objektbereich des Operationsmikroskops.

Fig. 7 und Fig. 8          modifizierte Ausführungsformen für ein OCT-System in dem Operationsmikroskop.

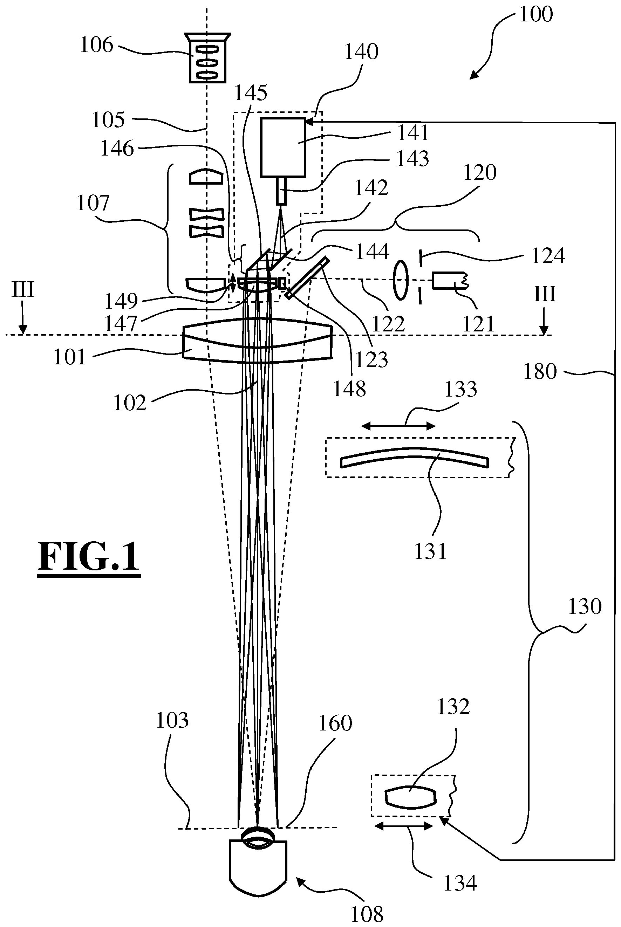

Das Operationsmikroskop 100 in Fig. 1 hat ein Mikroskop-Hauptobjektiv 101 mit einer optischen Achse 102 sowie einer Fokusebene 103. Das Mikroskop-Hauptobjektiv 101 wird von stereoskopischen Beobachtungsstrahlengängen 105 eines Binokulartubus 106 durchsetzt.

Zur Beleuchtung des Objektbereichs in Form eines Patientenauges 108 hat das Operationsmikroskop 100 als Beleuchtungseinrichtung ein Beleuchtungsmodul 120. Dieses Beleuchtungsmodul 120 umfasst einen ersten Lichtleiter 121, der Beleuchtungslicht 122 von einer nicht weiter dargestellten Lichtquelle bereitstellt. Mit dem aus dem Lichtleiter 121 austretenden Beleuchtungslicht 122 wird eine verstellbare Leuchtfeldblende 124 ausgeleuchtet. Mit einem Umlenkspiegel 123, der auf der objektabgewandten Seite des Mikroskop-Hauptobjektivs 101 angeordnet ist, wird das aus dem Lichtleiter 121 austretende Beleuchtungslicht durch das Mikroskop-Hauptobjektiv 101 hindurch in den Objektbereich 108 gelenkt.

Dem Ophthalmo-Operationsmikroskop 100 ist ein Ophthalmoskopie-Vorsatzmodul 130 mit einer Reduzierlinse 131 und einer Ophthalmoskopierlupe 132 zugeordnet, die in den stereoskopischen Beobachtungsstrahlengang 105 des Operationsmikroskops 100 entsprechend der Doppelpfeile 133, 134 ein- und ausgeschwenkt werden können.

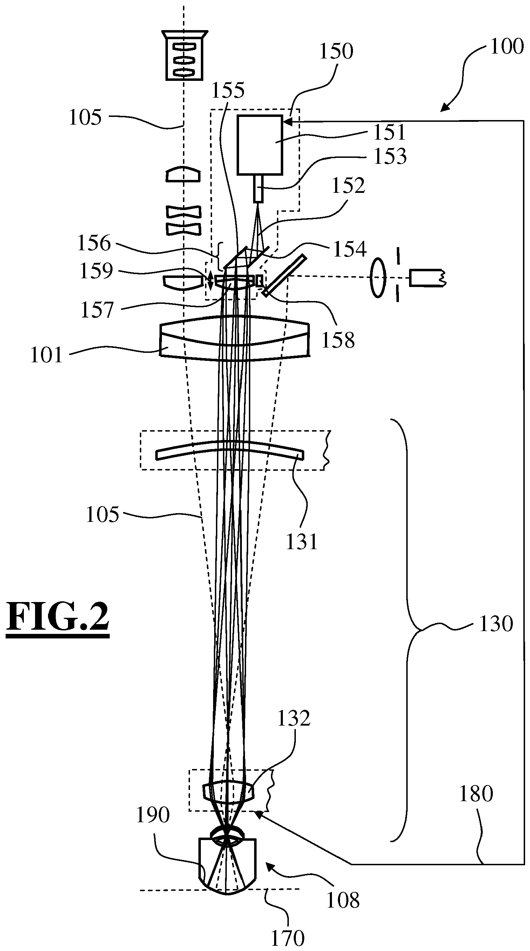

In dem Ophthalmo-Operationsmikroskop 100 ist ein erstes OCT-System 140 vorgesehen. Das Ophthalmo-Operationsmikroskop enthält weiter ein zweites OCT-System, das in Fig. 2 gezeigt ist. Diese OCT-Systeme ermöglichen die Aufnahme von OCT-Bildern.

Das OCT-System 140 aus Fig. 1 umfasst eine Einheit 141 für die Erzeugung und Analyse eines OCT-Abtaststrahlengangs 142. Die Einheit 141 ist in das Operationsmikroskop 100 integriert. Sie kann aber auch außerhalb des Operationsmikroskops 100 in einer entsprechenden Stativkonsole angeordnet werden. Die Einheit 141 ist mit einem Lichtleiter 143 verbunden, der den OCT-Abtaststrahlengang 142 bereitstellt.

Der aus dem Lichtleiter 143 austretende OCT-Abtaststrahlengang 142 wird mit divergentem Strahlengang auf einen ersten Scanspiegel 144 und einen zweiten Scanspiegel 145 einer OCT-Scaneinheit 146 geführt. Von dort gelangt er zu einem Optikelement in Form einer Sammellinse 147, um darauf das Mikroskop-Hauptobjektiv 101 zu durchsetzen. Der OCT-Abtaststrahlengang 142 wird in einer OCT-Abtastebene 160 im Frontabschnitt des Patientenauges 108 gebündelt.

Das in den OCT-Abtaststrahlengang aus dem Objektbereich in Form eines Patientenauges 108 zurückgestreute OCT-Licht gelangt über das Mikroskop-Hauptobjektiv 101, die Sammellinse 147 und die OCT-Scaneinheit 146 zurück in die Einheit 141. Dort wird das aus dem Objektbereich zurückgestreute OCT-Abtastlicht mit OCT-Strahlung aus einem Referenzstrahlengang interferiert. Das Interferenzsignal wird mittels eines Detektors erfasst und durch eine Rechnereinheit ausgewertet, die aus diesem Signal eine optische Weglängendifferenz zwischen Streuzentren für OCT-Licht im Objektbereich und der Weglänge von Licht im Referenzzweig bestimmt.

Zur Einstellung der OCT-Abtastebene 160 ist der Sammellinse 147 ein Verstellmechanismus 148 zugeordnet, mittels dessen diese entsprechend dem Doppelpfeil 149 bewegt werden kann.

Das erste OCT-System 140 arbeitet bei einer Wellenlänge von $\lambda$ = 1310 $nm$. Das zweite OCT-System im Ophthalmo-Operationsmikroskop 100 ist entsprechend dem ersten OCT-System 140 aufgebaut, hat jedoch eine Arbeitswellenlänge von $\lambda$ = 800 $nm$. Selbstverständlich könnten die OCT-Systeme auch für andere Arbeitswellenlängen ausgelegt werden. Arbeitswellenlängen lassen sich insbesondere im Bereich 600nm < $\lambda$ < 1500nm realisieren und sind je nach Applikation vorteilhaft.

Die Fig. 2 zeigt das Ophthalmo-Operationsmikroskop 100 aus Fig. 1 mit dem zweiten OCT-System 150, das entsprechend dem ersten OCT-System 140 darin aufgebaut und angeordnet ist.

Identische Baugruppen des Operationsmikroskops sind dabei in Fig. 2 mit denselben Bezugszeichen wie in Fig. 1 versehen.

Das OCT-System 150 hat eine Einheit 151 zur Erzeugung und Analyse eines OCT-Abtaststrahlengangs 152 des OCT-Systems 150. Es generiert am Austrittsende eines Lichtleiters 153 OCT-Abtaststrahlung 152. Diese OCT-Abtaststrahlung 152 ist wie die OCT-Abtaststrahlung 142 des OCT-Systems 190 aus Fig. 1 durch eine OCT-Scaneinheit 156 mit erstem Scanspiegel 154 und zweitem Scanspiegel 155 über ein als Sammellinse 157 ausgebildetes Optikelement durch das Mikroskop-Hauptobjektiv 101 geführt.

Fig. 2 zeigt das Ophthalmo-Operationsmikroskop 100 in einem Betriebsmodus, bei dem die Reduzierlinse 131 und die Ophthalmoskopierlupe 132 des Ophthalmoskopie-Vorsatzmoduls 130 in den Beobachtungsstrahlengang 205 des Operationsmikroskops 100 eingeschwenkt ist. Dies ermöglicht eine Untersuchung des Hintergrundes 190 des Patientenauges 108 durch Licht, das vom Augenhintergrund 190 zurück in die Beobachtungsstrahlengänge des Operationsmikroskops gelangt und mit OCT-Abtastlicht.

Die Sammellinse 157 bündelt den OCT-Abtaststrahlengang und lenkt ihn zum Mikroskop-Hauptobjektiv 101. Durch das Mikroskop-Hauptobjektiv 101, die Reduzierlinse 131 und die Ophthalmoskopierlupe 132 gelangt die OCT-Abtaststrahlung in das Patientenauge 108 und wird in der OCT-Abtastebene 170 am Augenhintergrund 190 des Patientenauges 108 gebündelt.

Das in den OCT-Abtaststrahlengang aus dem Objektbereich in Form eines Patientenauges 108 zurückgestreute OCT-Licht wird über das Mikroskop-Hauptobjektiv 101, die Sammellinse 157 und die OCT-Scaneinheit 156 zurück in die Einheit 151 zur Erzeugung und Analyse eines OCT-Abtaststrahlengangs geführt. Dort wird das aus dem Objektbereich zurückgestreute OCT-Abtastlicht wiederum mit OCT-Strahlung aus einem Referenzstrahlengang interferiert. Wie bei dem OCT-System 140 wird auch im OCT-System 150 das Interferenzsignal mittels eines Detektors erfasst und durch eine Rechnereinheit ausgewertet, die aus diesem Signal eine optische Weglängendifferenz zwischen Streuzentren für OCT-Licht im Objektbereich und der Weglänge von Licht im Referenzzweig bestimmt.

Der Sammellinse 157 ist ein Verstellmechanismus 157 zugeordnet, mittels dessen diese entsprechend dem Doppelpfeil 159 bewegt werden kann. Auf diese Weise kann auch für die OCT-Abtaststrahlung aus dem OCT-System 150 eine Fokusebene eingestellt werden.

Die optische Weglänge des OCT-Abtaststrahlengangs in dem in Fig. 2 gezeigten Betriebsmodus des Ophthalmo-Operationsmikroskops 100 ist länger als in dem Betriebsmodus nach Fig. 1. Dies macht eine Anpassung der optischen Weglänge im Referenzstrahlengang des OCT-Systems 150 erforderlich. Dazu ist eine Kopplung 180 von Ophthalmoskopierlupe 132 und OCT-System 150 vorgesehen, die bewirkt, dass bei Einschwenken der Ophthalmoskopierlupe 132 in den Beobachtungs- und OCT-Strahlengang die optische Weglänge des Referenzstrahlenganges im OCT-System entsprechend einem bestimmten Wert vergrößert wird. Dieser Wert ist vorzugsweise einstellbar gehalten. Ein festgelegter Wert orientiert sich günstigerweise an der Durchschnittslänge eines Patientenauges.

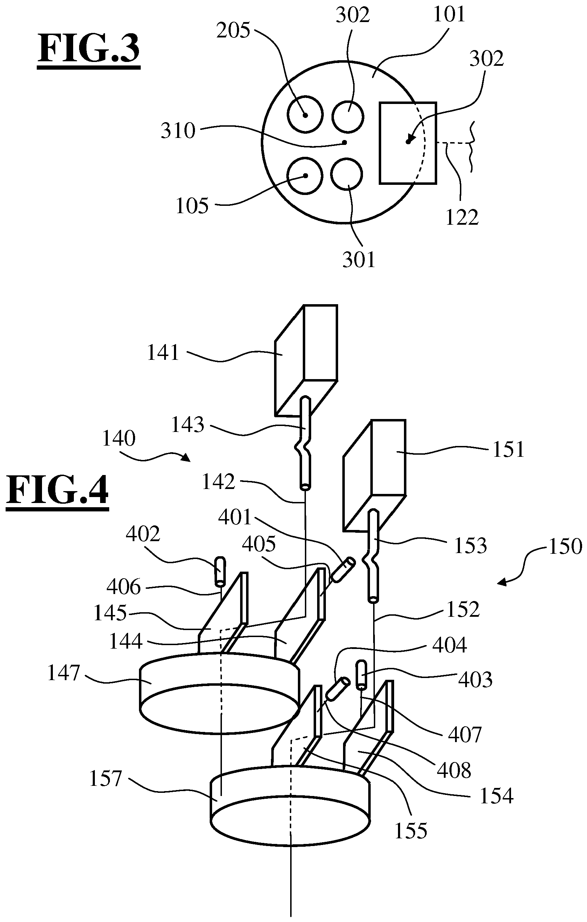

Fig. 3 ist ein Schnitt entlang der Linie III-III aus Fig. 1. Diese Figur erläutert den Verlauf der stereoskopischen Beobachtungsstrahlengänge 105, 205 des Operationsmikroskops 100 aus Fig. 1. Das Mikroskop-Hauptobjektiv 101 wird von zwei stereoskopischen Teilstrahlengängen 105, 205 durchsetzt. Die optische Achse 102 des Mikroskop-Hauptobjektivs liegt in dessen Zentrum 310. Der OCT-Abtaststrahlengang 142 des OCT-Systems 140 aus Fig. 1 durchsetzt das Mikroskop-Hauptobjektiv 101 im Bereich 301. Im Bereich 302 wird es vom OCT-Abtaststrahlengang 152 des OCT-Systems 150 aus Fig. 2 und im Bereich 303 von dem Beleuchtungslicht 122 durchsetzt.

Fig. 4 zeigt das erste OCT-System 140 und das zweite OCT-System 150 im Operationsmikroskop 100 aus Fig. 1 bzw. Fig. 2. Der Wellenlängenbereich der OCT-Abtaststrahlengänge der beiden OCT-Systeme 140, 150 ist allerdings unterschiedlich: Das erste OCT-System basiert auf OCT-Abtaststrahlung der Wellenlänge $\lambda_1 = 1310nm$. Das zweite OCT-System 320 arbeitet mit OCT-Abtaststrahlung der Wellenlänge $\lambda_2 = 800nm$. Zur Bezeichnung der Baugruppen der OCT-Systeme 140 und 150 in Fig. 4 sind die gleichen Bezugszeichen wie in Fig. 1 bzw. Fig. 2 verwendet.

Der erste Scanspiegel 144, 154 und der zweite Scanspiegel 145, 155 der OCT-Systeme 140, 150 sind mittels Stellantrieben 401, 402, 403, 404 um zwei zueinander senkrecht verlaufende Achsen 405, 406, 407 und 408 drehbeweglich angeordnet. Dies ermöglicht, die OCT-Abtaststrahlengänge 142, 152 unabhängig voneinander über eine Ebene zu scannen.

Der OCT-Abtaststrahlengang 142 des ersten OCT-Systems 140 wird über die Sammellinse 147 zu dem Mikroskop-Hauptobjektiv 101 geführt. Der OCT-Abtaststrahlengang 152 des zweiten OCT-Systems 150 gelangt über die Sammellinse 157 durch das Mikroskop-Hauptobjektiv 101.

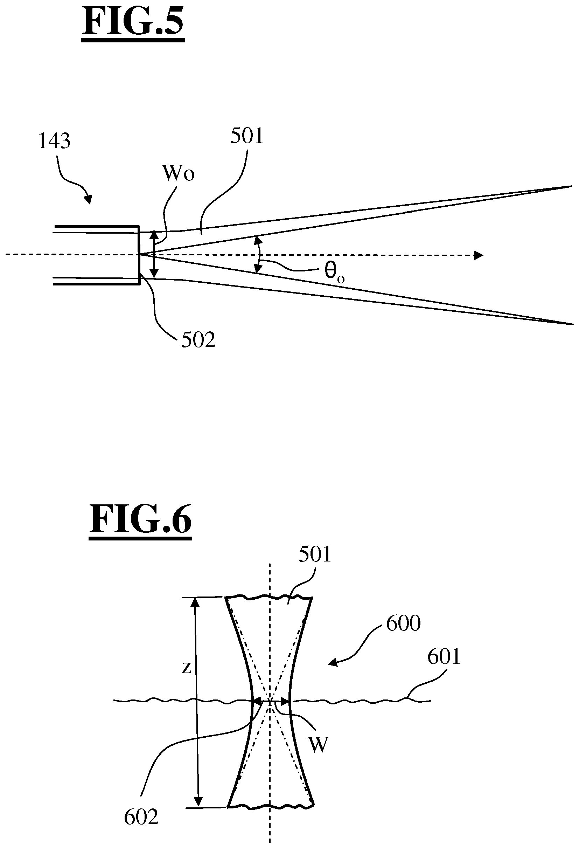

Fig. 5 zeigt den Frontabschnitt 502 des Lichtleiters 143 aus Fig. 1. Der Lichtleiter 143 wirkt für Licht der Wellenlänge $\lambda_1 = 1310nm$ als Monomodenfaser. Der Durchmesser $d_1$ des Faserkerns des Lichtleiters 143 genügt der Beziehung

$$\frac{d_1}{2} < 2.4 \frac{\lambda_1}{2\pi NA_1},$$

wobei $NA_1$ die numerische Apertur der Frontfläche des Lichtleiters ist. Vorzugsweise liegt der Durchmesser d des Faserkerns des Lichtleiters 122 im Bereich $5\mu m < d < 10\mu m$. In diesem Parameterbereich führt der Lichtleiter 143 das Licht mit gaussförmigen Wellenmoden. Aus dem Lichtleiter 143 tritt der OCT-Abtastlichtstrahl 501 mit einem näherungsweise gaussförmigen Strahlungsprofil aus, das durch einen Taillenparamter $W_1$ und einem Öffhungsparameter $\theta_1$ charakterisiert ist, wobei gilt:

$$\theta_1 = \frac{\lambda_1}{\pi W_1}$$

Für einen Faserkerndurchmesser von $d_1 = 10\mu m$, einen Wellenlänge $\lambda_1 = 1310nm$ ergibt sich damit als Maß für die Strahldivergenz ein Öffnungswinkel von $\theta_1 \approx 0{,}0827$ rad.

Die Frontfläche 502 des Lichtleiters 143 wird über die Scanspiegel 144 und 145 im Operationsmikroskop 100 aus Fig. 1, die Sammellinse 147 durch das Mikroskop-Hauptobjektiv in eine OCT-Abtastebene abgebildet.

Die Fig. 6 zeigt den Verlauf der Intensitätsverteilung des OCT-Abtastlichtstrahls 501 senkrecht zur OCT-Abtastebene 601. In der OCT-Abtastebene 601 hat die Intensitätsverteilung der OCT-Abtaststrahlung eine kleinste Einschnürung. Außerhalb der OCT-Abtastebene nimmt der Durchmesser des OCT-Abtaststrahlengangs zu. Da der OCT-Abtastlichtstrahl 501 aus dem Lichtleiter 143 aus Fig. 5 mit einem näherungsweise gaussförmigen Strahlungsprofil austritt, bewirken die Sammellinse 147 und das Mikroskop-Hauptobjektiv 101 des Operationsmikroskops 100 aus Fig. 1 für den OCT-Abtastlichtstrahl im Bereich der OCT-Abtastebene 160 ein sogenanntes Gaussbündel 600 des OCT-Abtastlichtstrahls 501. Dieses Gaussbündel 600 ist durch den konfokalen Parameter $z_1$ als Maß für die longitudinale Ausdehnung der Taille des Gaussbündels, sowie den Taillenparameter $W_{1,A}$ als Mass für den Durchmesser der kleinsten Einschnürung 602 des OCT-Abtastlichtstrahls 501 in der OCT-Abtastebene, d.h. für den Durchmesser von dessen Taille charakterisiert, wobei gilt:

$$z_1 = 2\frac{W_{1,A}^2\,\pi}{\lambda_1},$$

dabei ist $\lambda_1$ die Wellenlänge des OCT-Abtastlichtstrahls. Zwischen dem Taillenparametern $W_{1,A}$ des Gaussbündels 600 und dem Taillenparameter $W_1$ des in Fig. 5 gezeigten, aus dem Lichtleiter 143 austretenden Abtastlichtstrahls 501 gilt die folgende Beziehung:

$$W_{1,A} = \beta_1 W_1,$$

wobei $\beta_1$ der Vergrößerungs- bzw. Verkleinerungsparameter der oben erwähnten geometrischen Abbildung des Austrittsendes von Lichtleiter 143 aus Fig. 1 in der OCT-Abtastebene ist. $\beta_1$ ist mit der Brennweite $f_{147}$ der Sammellinse 147 aus Fig. 1 und der Brennweite $f_2$ des Mikroskop-Hauptobjektivs über die folgende Beziehung verknüpft:

$$\frac{f_2}{f_{147}} = \beta_1$$

Die Größe von Strukturen, die sich mit dem OCT-Abtastlichtstrahl 501 auflösen lassen, ist durch dessen Durchmesser in der OCT-Abtastebene 601, d.h. durch den Taillenparameter $W_1$ bestimmt. Erfordert beispielsweise eine Applikation eine Lateralauflösung des OCT-Systems im Operationsmikroskop von ca. 40 $\mu$m, muss nach dem Nyquist-Theoren der Querschnitt des OCT-Abtastlichtstrahls 501 in der OCT-Abtastebene ca. 20 $\mu$m betragen. Bei gegebener Wellenlänge $\lambda_1$ für den OCT-Abtastlichtstrahl 143 aus Fig. 1 müssen daher für eine gewünschte Auflösung des OCT-Systems 140 die Vergrößerung der optischen Abbildung im OCT-Strahlengang und der Durchmesser des Faserkerns im Lichtleiter 143 geeignet gewählt werden.

Der konfokale Parameter $z_1$ als Maß für die longitudinale Ausdehnung der Taille des Gaussbündels bestimmt den axialen Tiefenbereich, aus dem in dem OCT-Abtaststrahlengang 143 aus Fig. 1 zurückgestreutes Licht detektiert werden kann: Je kleiner der konfokale Parameter $z_1$, desto größer ist der Verlust des OCT-Systems an lateraler Auflösung bei Entfernung eines im OCT-Abtaststrahlung abgetasteten Objekts von der OCT-Abtastebene 601, da sich der Ort von Streuzentren nur innerhalb des durch den Taillenparamter $W_1$ und den konfokalen Parameter $z_1$ definierten "Trichter" lokalisieren lässt.

Nachdem die axiale Auflösung eines OCT-Systems einerseits durch die Kohärenzlänge $l_c$ des Lichts der im OCT-System eingesetzten Lichtquelle begrenzt ist, andererseits die laterale Auflösung des OCT-Systems abnimmt, wenn dessen Tiefenhub den konfokalen Parameter übersteigt, ist das Einstellen des konfokalen Parameters $z_1$ auf die Kohärenzlänge $l_c$ der im OCT-System eingesetzten Lichtquelle günstig.

Für eine bestimmte Wellenlänge $\lambda_1$ des OCT-Abtastlichtstrahls 501 ergibt sich dann die mögliche laterale Auflösung des OCT-Systems aus Fig. 1, da die Wellenlänge $\lambda_1$ und der Rayleighparameter $z_1$ den Taillenparameter $W_{1,A}$ festlegen. Die Optikeinheiten im OCT-Abtaststrahlengang 143 aus Fig. 1 und die Bemassung des Faserkerns von Lichtleiter 143 sind dann so zu wählen, dass der betreffende Taillenparamter realisiert wird.

Die Sammellinse 147 im Operationsmikroskop 100 ist vorzugsweise so eingestellt, dass die Fokusebene 170 des Mikroskop-Hauptobjektivs 101 für den sichtbaren Spektralbereich und die OCT-Abtastebene 160 des OCT-Systems 140 zusammenfallen. Dann liegt die in Fig. 5 gezeigte Taille 502 des OCT-Abtaststrahls in der Fokusebene des Operationsmikroskops. Für den OCT-Abtaststrahlengang 152 des OCT 150 aus Fig. 2 wirkt der Lichtleiter 153 für Licht der Wellenlänge $\lambda_2 = 800\,nm$ als Monomodenfaser. Der Durchmesser $d_2$ des Faserkerns des Lichtleiters 153 genügt deshalb der Beziehung:

$$\frac{d_2}{2} < 2.4\frac{\lambda_2}{2\,\pi NA_2},$$

wobei $NA_2$ die numerische Apertur der Frontfläche des Lichtleiters 153 ist. Aus dem Lichtleiter 153 tritt dass wiederum der OCT-Abtastlichtstrahl 152 mit einem näherungsweise gaussförmigen Strahlungsprofil aus, das durch einen Taillenparameter $W_2$ und einen Öffhungsparameter $\theta_2$ charakterisiert ist:

$$\theta_2 = \frac{\lambda_2}{\pi W_2}$$

Für den Taillenparamter des OCT-Abtastlichtstrahls 152 in der OCT-Abtastebene 170 gilt damit:

$$W_{2,A} = \beta_2 W_2 \, ,$$

wobei $\beta_2$ der Vergrößerungs- bzw. Verkleinerungsparameter der geometrischen Abbildung des Austrittsendes von Lichtleiter 153 aus Fig. 2 in der OCT-Abtastebene 170 ist.

$\beta_2$ ist durch die Brennweite der Sammellinse 157 aus Fig. 2, die Brennweite des Mikroskop-Hauptobjetkivs 101 sowie durch die Reduzierlinse 131, die Ophthalmoskopierlupe 132 und die Cornea und Linse des Patientenauges 108 festgelegt.

Die Sammellinse 157 ist dabei vorzugsweise so eingestellt, dass, wenn der optische Beobachtungsstrahlengang durch das Mikroskop-Hauptobjektiv 101 den Augenhintergrund 190 des Patientenauges 108 abbildet, die OCT-Abtastebene 170 für das OCT-System 150 im Operationsmikroskop 100 mit dem Augenhintergrund 190 zusammenfällt.

Alternativ zu der beschriebenen Auslegung der OCT-Systeme im Operationsmikroskop kann auch ein Versatz von OCT-Abtastebene und Fokusebene im Operationsmikroskop vorgesehen sein. Vorzugsweise ist dieser Versatz nicht größer als der konfokale Parameter z des OCT-Abtastlichtstrahls im Bereich der OCT Abtastebene.

Indem die OCT-Abtastebene um den konfokale Parameter z weiter vom Mikroskop-Hauptobjektiv 101 aus Fig. 1 entfernt ist, lässt sich der Tiefenhub für das OCT-System im Objektbereich maximieren.

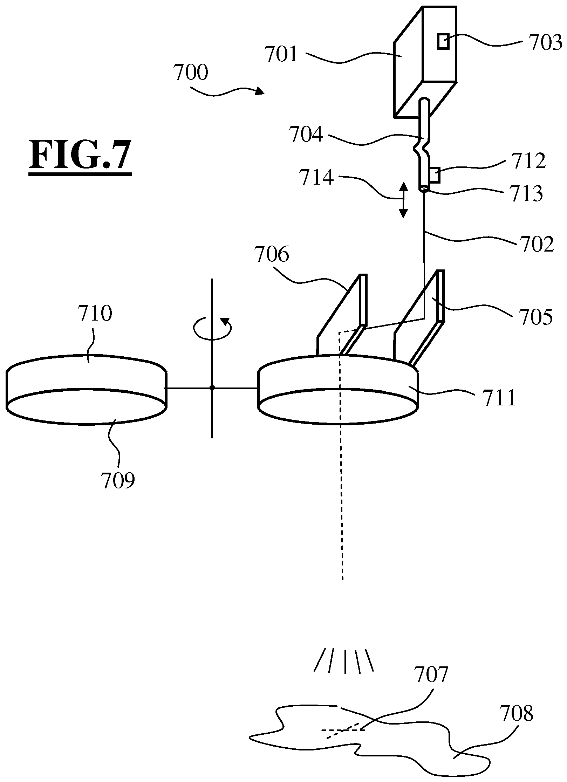

In Fig. 7 ist eine modifizierte Ausführungsform für ein OCT-System gezeigt, das in dem Ophthalmo-Operationsmikroskop 100 nach Fig. 1 und Fig. 2 eingesetzt werden kann.

Das OCT-System 700 umfasst entsprechend dem OCT-System 140 aus Fig. 1 eine Einheit 701 für die Erzeugung und Analyse eines OCT-Abtaststrahlengangs 702. Die Einheit 701 ist dabei so ausgelegt, dass OCT-Abtaststrahlengänge mit unterschiedlicher Wellenlänge erzeugt und ausgewertet werden können. Für das Einstellen der Wellenlänge des OCT-Systems 700 ist ein Steuergerät 703 vorgesehen.

Der OCT-Abtaststrahlengang 702 tritt aus einem Lichtleiter 704 aus, der mit der Einheit 701 verbunden ist. Er gelangt zu zwei Scanspiegeln 705, 706 die mittels nicht weiter dargestellten Antrieben derart beweglich sind, dass in einer OCT-Abtastebene 707 ein Objektbereich 708 abgetastet werden kann.

Für die Einstellung unterschiedlicher OCT-Abtastebenen ist bei dem OCT-System 700 einerseits ein Vergrößerungswechsler 709 als Linsenwechseleinrichtung vorgesehen, der Sammellinsen unterschiedlicher Brechkraft 710, 711 enthält. Diese Sammellinsen 710, 711 können in den OCT-Abtaststrahlengang 702 ein- und ausgeschwenkt werden. Weiter ist dem Lichtleiter 704 eine Antriebseinheit 712 zugeordnet, die es ermöglicht, dass Lichtleiter-Austrittsende 713 entsprechend der Doppelpfeile 714 zu bewegen, um auf diese Weise die Position der OCT-Abtastebene 707 im Objektbereich variieren zu können.

In die OCT-Abtastebene gelangt der OCT-Abtaststrahlengang 707 durch die Sammellinse 709, welche den Abtaststrahlengang bündelt und zum Mikroskop-Hauptobjektiv des entsprechenden Operationsmikroskops weiterleitet.

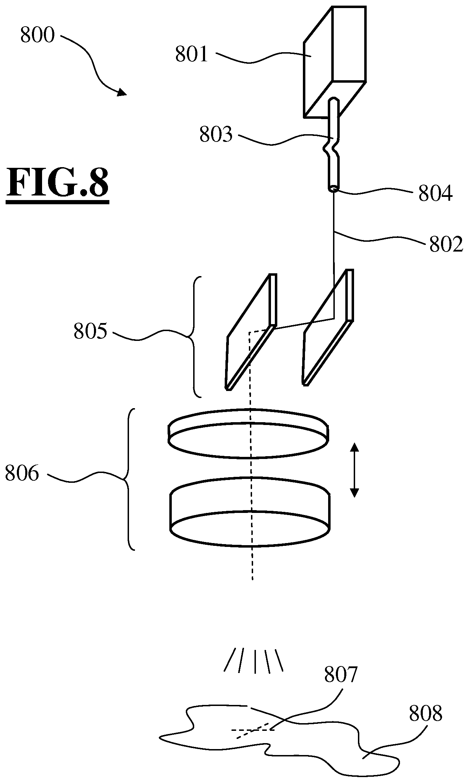

Fig. 8 zeigt eine weitere Ausführungsform eines OCT-Systems für ein Ophthalmo-Operationsmikroskop. Das OCT-System hat wie das OCT-System 700 eine Einheit 801 für die Erzeugung und Analyse eines OCT-Abtaststrahlengangs 802, der aus dem einen Lichtleiter 803 austritt. In dem OCT-System 800 wird der aus dem Lichtleiter-Austrittende 804 austretende OCT-Abtaststrahlengang 802 über ein entsprechendes Scanspiegelsystem 805 durch eine Linseneinheit 806 mit einstellbarer Brechkraft geführt, welche als Zoomsystem wirkt. So ist es wiederum möglich, die Position der OCT-Abtastebene 807 in einem Objektbereich 808 zu variieren.

Eine weitere modifizierte Ausführungsform des anhand von Fig. 1 erläuterten Operationsmikroskops 100 enthält ein fokussierbares Mikroskop-Hauptobjektiv mit einstellbarer Brennweite. Auch diese Maßnahme ermöglicht das Verlagern einer OCT-Abtastebene und das Verändern der geometrischen Abbildung des Lichtleiter-Austrittsendes in die OCT-Abtastebene.

**Patentansprüche**

1. Ophthalmo-Operationsmikroskop,

   - mit einem Mikroskop-Hauptobjektiv (101);
   - mit einem Beobachtungsstrahlengang (105, 205), der zur Visualisierung eines Objektbereichs (108) das Mikroskop-Hauptobjektiv (101) durchsetzt; und

- mit einem OCT-System (140, 150, 700, 800) zur Aufnahme von Bildern des Objektbereichs (108), wobei das OCT-System (140, 150, 700, 800) einen OCT-Abtaststrahlengang (142, 152, 702, 802) umfasst, der über eine Scanspiegelanordnung (146, 156, 705, 706, 805) zum Objektbereich (108) geführt ist;

**dadurch gekennzeichnet, dass**

- ein Ophthalmoskopie-Vorsatzmodul (130) mit Ophthalmoskopierlupe (132) und Reduzierlinse (131) vorhanden ist, das in den Beobachtungsstrahlengang (105, 205) und den OCT-Abtaststrahlengang (142, 152) ein- und ausgeschwenkt werden kann, und dass

- eine Kopplung von OCT-System (140) und Ophthalmoskopierlupe (132) in der Weise vorhanden ist, dass bei Einschwenken der Ophthalmoskopierlupe (132) die optische Weglänge im Referenzstrahlengang des OCT-Systems um einen bestimmten, einstellbar gehaltenen Wert vergrößert wird.

2. Opthalmo-Operationsmikroskop nach Anspruch 1, **dadurch gekennzeichnet, dass**

- zwischen der Scanspiegelanordnung (146, 156, 705, 706, 805) und dem Mikroskop-Hauptobjektiv (101) ein Optikelement (147, 157, 711, 806) vorhanden ist, das den aus der Scanspiegelanordnung (146, 156, 705, 706, 805) austretenden OCT-Abtaststrahlengang (142, 152, 702, 802) bündelt und in einen Strahlengang überführt, der das Mikroskop-Hauptobjektiv (101) durchsetzt.

3. Ophthalmo-Operationsmikroskop nach Anspruch 2, **dadurch gekennzeichnet, dass** das Optikelement als bewegliche Linseneinheit (147, 157) ausgebildet ist.

4. Ophthalmo-Operationsmikroskop nach Anspruch 2 oder 3, **dadurch gekennzeichnet, dass** das Optikelement (711) in einer Linsenwechseleinrichtung (709) aufgenommen ist.

5. Ophthalmo-Operationsmikroskop nach Anspruch 2, **dadurch gekennzeichnet, dass** das Optikelement als Zoomsystem (806) mit variabler Brennweite ausgebildet ist.

6. Ophthalmo-Operationsmikroskop nach einem der Ansprüche 2 bis 5, **dadurch gekennzeichnet, dass** Mittel zur Einstellung der Brennweite des Mikroskop-Hauptobjektivs vorhanden sind.

7. Ophthalmo-Operationsmikroskop nach einem der Ansprüche 2 bis 6, **dadurch gekennzeichnet, dass** die Scanspiegelanordnung (146, 156) für das Scannen des OCT-Abtaststrahlengangs einen ersten Scanspiegel (144, 154) umfasst, der um eine erste Drehachse (301, 303) bewegt werden kann.

8. Ophthalmo-Operationsmikroskop nach Anspruch 7, **dadurch gekennzeichnet, dass** ein zweiter Scanspiegel (145,155) vorhanden ist, der um eine zweite Drehachse (302, 304) bewegt werden kann, wobei die erste Drehachse (301, 303) und die zweite Drehachse (302, 304) seitlich versetzt in einem rechten Winkel zueinander stehen.

9. Ophthalmo-Operationsmikroskop nach einem der Ansprüche 2 bis 8. **dadurch gekennzeichnet, dass** das das OCT-System einen Lichtleiter (704) umfasst, der einen Lichtaustrittsabschnitt (713) für den OCT-Abtaststrahlengang aufweist, wobei Mittel (712) zum Bewegen des Lichtaustrittsabschnitts (713) des Lichtleiters (704) vorgesehen sind.

## Claims

1. Ophthalmic surgical microscope,

- having a microscope main objective (101);
- having an observation beam path (105, 205) which passes through the microscope main objective (101) for visualizing an object region (108); and
- having an OCT system (140, 150, 700, 800) for recording images of the object region (108), wherein the OCT system (140, 150, 700, 800) comprises an OCT scanning beam path (142, 152, 702, 802) which is guided via a scanning mirror arrangement (146, 156, 705, 706, 805) to the object region (108);

**characterized in that**

- an ophthalmoscopy attachment module (130) with an ophthalmoscopy magnifier (132) and a reduction lens (131) is present, said ophthalmoscopy attachment module being able to be pivoted into and out of the observation beam path (105, 205) and the OCT scanning beam path (142, 152), and **in that**
- there is a coupling of OCT system (140) and ophthalmoscopy magnifier (132) in such a way that, when the ophthalmoscopy magnifier (132) is pivoted-in, the optical path length in the reference beam path of the OCT system is increased by a certain, adjustably kept value.

2. Ophthalmic surgical microscope according to Claim 1, **characterized in that**

- an optical element (147, 157, 711, 806) is present between the scanning mirror arrangement (146, 156, 705, 706, 805) and the microscope main objective (101), said optical element focusing the OCT scanning beam path (142, 152, 702, 802) emerging from the scanning mirror arrangement (146, 156, 705, 706, 805) and transforming the latter into a beam path which passes through the microscope main objective (101).

3. Ophthalmic surgical microscope according to Claim 2, **characterized in that**
the optical element is embodied as a movable lens unit (147, 157).

4. Ophthalmic surgical microscope according to Claim 2 or 3, **characterized in that**
the optical element (711) is received in a lens interchange device (709).

5. Ophthalmic surgical microscope according to Claim 2, **characterized in that**
the optical element is embodied as a zoom system (806) with a variable focal length.

6. Ophthalmic surgical microscope according to any one of Claims 2 to 5,
**characterized in that** means for adjusting the focal length of the microscope main objective are present.

7. Ophthalmic surgical microscope according to any one of Claims 2 to 6,
**characterized in that** the scanning mirror arrangement (146, 156) comprises a first scanning mirror (144, 154) for scanning the OCT scanning beam path, said first scanning mirror being movable about a first axis of rotation (301, 303).

8. Ophthalmic surgical microscope according to Claim 7, **characterized in that**
a second scanning mirror (145, 155) is present, said second scanning mirror being able to be moved about a second axis of rotation (302, 304), wherein the first axis of rotation (301, 303) and the second axis of rotation (302, 304) are laterally offset at right angles to one another.

9. Ophthalmic surgical microscope according to any one of Claims 2 to 8,
**characterized in that** the OCT system comprises a light guide (704) which has a light-emergence section (713) for the OCT scanning beam path, wherein means (712) are provided for moving the light-emergence section (713) of the light guide (704).

**Revendications**

1. Microscope d'opération ophtalmologique, comportant

- un objectif principal de microscope (101) ;
- un chemin de faisceau d'observation (105, 205) qui traverse l'objectif principal de microscope (101) pour visualiser une zone objet (108) ; et
- un système OCT (140, 150, 700, 800) destiné à acquérir des images de la zone objet (108), dans lequel le système OCT (140, 150, 700, 800) comprend un chemin de faisceau d'échantillonnage OCT (142, 152, 702, 802) qui est guidé par l'intermédiaire d'un dispositif à miroirs de balayage (146, 156, 705, 706, 805) vers la zone objet (108) ;

**caractérisé en ce qu'**il est prévu

- un module adaptateur ophtalmoscopique (130), comportant une loupe ophtalmoscopique (132) et une lentille réductrice (131), qui peut être introduit dans le faisceau d'observation (105, 205) et le chemin de faisceau d'échantillonnage OCT (142, 152) et en être sorti, et **en ce qu'**il est prévu
- un couplage entre le système OCT (140) et la loupe ophtalmoscopique (132) de manière à ce que, lorsque la loupe ophtalmoscopique (132) est introduite dans le faisceau, la longueur de trajet optique sur le chemin du faisceau de référence du système OCT est augmentée d'une valeur déterminée qui est maintenue réglable.

2. Microscope d'opération ophtalmologique selon la revendication 1, **caractérisé en ce qu'**il est prévu :

   - entre le dispositif à miroirs de balayage (146, 156, 705, 706, 805) et l'objectif principal de microscope (101) un élément optique (147, 157, 711, 806) qui focalise le chemin de faisceau d'échantillonnage OCT (142, 152, 702, 802) sortant du dispositif à miroirs de balayage (146, 156, 705, 706, 805) et le transfère sur un chemin de faisceau passant à travers l'objectif principal de microscope (101).

3. Microscope d'opération ophtalmologique selon la revendication 2, **caractérisé en ce que** l'élément optique est réalisé sous la forme d'une unité à lentille mobile (147, 157).

4. Microscope d'opération ophtalmologique selon la revendication 2 ou 3, **caractérisé en ce que** l'élément optique (711) est logé dans un moyen d'échange de lentilles (709).

5. Microscope d'opération ophtalmologique selon la revendication, **caractérisé en ce que** l'élément optique est réalisé sous la forme d'un système de zoom (806) à focale variable.

6. Microscope d'opération ophtalmologique selon l'une quelconque des revendications 2 à 5, **caractérisé en ce qu'**il est prévu des moyens destinés à régler la longueur focale de l'objectif principal de microscope.

7. Microscope d'opération ophtalmologique selon l'une quelconque des revendications 2 à 6, **caractérisé en ce que** le dispositif à miroirs de balayage (146, 156) comprend, pour animer d'un mouvement de balayage le chemin de faisceau d'échantillonnage OCT, un premier miroir de balayage (144, 154) qui peut être déplacé autour d'un premier axe de rotation (301, 303).

8. Microscope d'opération ophtalmologique selon la revendication 7, **caractérisé en ce qu'**il est prévu un deuxième miroir de balayage (145, 155) qui peut être déplacé autour d'un deuxième axe de rotation (302, 304), dans lequel le premier axe de rotation (301, 303) et le deuxième axe de rotation (302, 304) sont décalés latéralement l'un par rapport à l'autre en formant un angle droit.

9. Microscope d'opération ophtalmologique selon l'une quelconque des revendications 2 à 8, **caractérisé en ce que** le système OCT comprend un guide optique (704) qui présente une section de sortie de lumière (713) destinée au chemin de faisceau d'échantillonnage OCT, dans lequel il est prévu des moyens (712) destinés à déplacer la section de sortie de lumière (713) du guide optique (704).

**FIG.1**

**FIG.2**

**FIG.3**

**FIG.4**

# FIG.5

143
501
Wo
$\theta_o$
502

# FIG.6

501
600
601
z
W
602

**FIG.7**

**FIG.8**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0697611 A2
- US 5321501 A
- WO 200610544 A1

- EP 1326117 A1
- EP 0815801 A2
- EP 0815801 A